# EUROPÄISCHE PATENTSCHRIFT

(11) **EP 1 673 068 B1**
(45) Veröffentlichungstag und Bekanntmachung des Hinweises auf die Patenterteilung: **02.12.2009**
(21) Anmeldenummer: 04766408.1
(22) Anmeldetag: 04.08.2004
(51) Int. Cl.: A61K 8/14, A61K 8/22, A61K 8/31, A61K 8/68, A61K 9/00, A61K 9/127, A61Q 19/00

(54) **KOSMETISCHE ZUSAMMENSETZUNG ZUR UNTERSTÜTZUNG DES SAUERSTOFFTRANSPORTS IN DIE HAUT**
COSMETIC COMPOSITION PROMOTING OXYGEN TRANSPORT INTO THE SKIN
COMPOSITION COSMETIQUE FAVORISANT LE TRANSPORT D'OXYGENE DANS LA PEAU

(30) Priorität: 11.08.2003 DE 10336841
(43) Veröffentlichungstag der Anmeldung: 28.06.2006
(73) Patentinhaber: ROVI Cosmetics International GmbH, 36381 Schlüchtern (DE)
(72) Erfinder: BLUME, Gabriele, 36396 Steinau a.d. Strasse (DE); SACHER, Michael, 36381 Schlüchtern (DE); TEICHMÜLLER, Dirk, 63589 Linsengericht (DE)
(74) Vertreter: Weber, Roland
(86) Internationale Anmeldenummer: PCT/EP2004/051702
(87) Internationale Veröffentlichungsnummer: WO 2005/016309

(56) Entgegenhaltungen:
- EP-A- 0 647 131
- EP-A- 0 665 002
- EP-A- 1 051 979
- WO-A-95/20945
- DE-A- 4 127 442
- DE-A- 19 810 999
- FR-A- 2 627 385
- US-A- 5 705 187

## Beschreibung

Die Erfindung betrifft eine kosmetische Zusammensetzung zur Unterstützung des Sauerstofftransports in die Haut, wobei die Züsammensetzung für den Sauerstofftransport einen Anteil an membranbildenden Lipiden und einen Anteil an Flüorcarbon oder Flüorcarbongemisch enthält. Der Begriff "kosmetische" Zusammensetzung umfasst im Sinne der vorliegenden Erfindung auch "pharmazeutische" Zusammensetzungen, d. h. Zusammensetzungen, die unter Arzneimittelrecht fallen.

Molekularer Sauerstoff spielt eine wichtige Rolle bei der Bereitstellung von Energie für eine Vielzahl von Prozessen, die in den Zellen höherer Organismen stattfinden. Nach der Aufnahme von Sauerstoff, z.B. über die Lungen, wird Sauerstoff an die Erythrozyten gebunden und über arterielle Blutgefäße und kleine Kapillargefäße durch den Körper an ihren Zielort transportiert. Dort wird der Sauerstoff an das Gewebe abgegeben und über die Atmungskette in den Mitochondrien unter Erhalt von Energie umgesetzt. Auch durch die Haut (transkutan) wird Sauerstoff aufgenommen und zu darunterliegenden Geweben transportiert. Beim Menschen wird häufig ab einem Alter von etwa 20 Jahren eine Abnahme des transkutanen Sauerstoffdrucks beobachtet. Diese örtliche Abnahme des Sauerstoffdrucks geht mit einem verminderten Austausch zwischen Wasser im Blutplasma und dem extrazellulären Fluid einher, wobei das subkutane Gewebe an Feuchtigkeit verliert und verminderte Mengen an Nährstoffen erhält. Die Diffusionskapazität der Kapillaren in diesem Bereich nimmt ab.

Dieser Sauerstoffmangel kann dazu führen, daß die Haut, insbesondere die Gesichtshaut, ihr junges und gesundes Erscheinungsbild verliert. Man spricht von frühzeitgem Altern der Gesichtshaut, was mit erhöhter Faltenbildung einhergeht. Die kosmetische Industrie bietet eine Vielzahl von Präparaten an, die einer solchen frühzeitigen Hautalterung und Faltenbildung entgegenwirken sollen. Einige dieser Präparate sollen neben einer feuchtigkeitsspendenden Wirkung molekularen Sauerstoff in das Unterhautgewebe transportieren und damit die mit höherem Alter zunehmende Unterversorgung dieses Gewebes mit Sauerstoff ausgleichen. Die verschiedenen auf dem Markt erhältlichen Präparate zeigen dabei eine mehr oder weniger zufriedenstellende Wirkung.

Die Verwendung von Fluorcarbonen als sauerstoffbindende Mittel in kosmetischen und medizinischen Präparaten ist bekannt. Die US-A-4,366,169 beschreibt die Verwendung von Fluorcarbonen zur Behandlung von Hautverletzungen und Wunden, insbesondere von Verbrennungen, wobei das Sauerstoff enthaltende Fluorcarbon entweder direkt oder als Emulsion auf die Haut, auf entsprechende Verbände oder ähnliche Mittel gebracht wird. Die US-A-4,569,784 beschreibt die Herstellung eines Gels mit Gastransporteigenschaften für die Anwendung auf der Haut. Das Verfahren besteht darin, daß eine mit Wasser nicht mischbare organische Flüssigkeit, z.B. ein Fluorcarbon, in Gegenwart eines Emulgators emuliert wird. Daran schließt sich ein Konzentrierungsprozeß an, der zur Bildung einer Gel-Phase führt. In dem darauffolgenden Schritt wird die Trennung der klaren Flüssigkeit von dem pastösen Feststoff (Gel-Phase) durch Dekantieren, Filtrieren oder Verdampfen bewirkt Das Gel wird in geeigneten Formulierungen auf der Haut angewendet und wirkt, ohne jedoch das Stratum Comeum der Haut zu durchdringen. Die EP-A-296 661 beschreibt ein Fluorcarbon enthaltendes Einphasensystem, das als isotrope oder anisotrope Formulierung Im kosmetischen Bereich und auch als Dermatikum als Sauerstofftransporteur wirken kann. Dabei werden Fluorcarbone mit einer maximalen Konzentration von 50% mit perfluorierten Emulgatoren vom Alkansulfonsäureamid-Typ in Gegenwart eines aliphatischen Alkohols als Hilfsemulgator in Wasser emulgiert. Die WO-A-8908459 beschreibt eine Perfluorcarbonemulsion mit Phospholipidvesikeln als Blutersatzstoff, bei die Phospholipidmonomere polymerisiert werden. Die WO-A-9100110 offenbart Fluorcarbonemulsionen mit Phospholipiden, bei denen das Phospholipid gesättigte Kohlenstoffbindungen hat. Aus der WO-A-9206676 sind mit Öl gefüllte Vesikel aus Phospholipiden bekannt, deren Struktur der üblichen Vesikelstruktur entspricht. Die EP-A-0 647 131 beschreibt ein Dermatikum für den Sauerstofftransport in der Haut, das asymmetrische lamellare Aggregate enthält, bestehend aus Phospholipiden mit einem Phosphatidylcholingehalt von 30-99 Gew.-% und mit Sauerstoff beladenem Fluorcarbon oder Fluorcarbongemisch, wobei die Aggregate eine Hautpenetrierung in Abhängigkeit von der kritischen Löslichkeitstemperatur in n-Hexan der ausgewählten Fluorcarbone oder Fluorcarbongemische haben.

Die meisten der bekannten Zusammensetzungen, soweit sie für den Transport von molekularem Sauerstoff in die Haut vorgesehen sind, haben den Nachteil, daß sie nicht in der Lage sind, das Stratum Comeum der Haut und die Epidermis zu überwinden und den molekularen Sauerstoff in das daran angrenzende Gewebe in ausreichender Menge zu transportieren.

Der vorliegenden Erfindung liegt daher die Aufgabe zugrunde, diesen Nachteil der aus dem Stand der Technik bekannten Zusammensetzungen zu überwinden und eine kosmetische Zusammensetzung bereitzustellen, die den Transport von molekularem Sauerstoff in die Haut, durch das Stratum Comeum der Haut und die Epidermis und bis in das angrenzende Gewebe unterstützt und dadurch die Sauerstoffkonzentration in dem Gewebe erhöht und Stoffwechselvorgänge aktiviert.

Erfindungsgemäß wird diese Aufgabe durch eine kosmetische Zusammensetzung der eingangs genannten Art gelöst, die 1 bis 50 Gew.-% membranbildende Sphingolipide und/oder Galactolipide und 5 bis 50 Gew.-% mit Sauerstoff beladenes Fluorcarbon oder Fluorcarbongemisch enthält, wobei die Sphingolipide und/oder Galactolipide als Transportvesikel für das mit Saüerstoff beladene Flüorcarbon oder Flüorcarbongemisch dienen. Es hat sich überraschenderweise gezeigt, daß die Verwendung von membranbildenden Sphingolipiden und/oder Galactolipiden als Transportvesikel bzw. zur Bildung von Transportvesikeln für mit Sauerstoff beladenes Fluorcarbon oder Fluorcarbongemisch einen ausgezeichneten und gegenüber bekannten Transportsystemen überragenden Sauerstofftransport durch das Stratum Comeum der Haut und die Epidermis liefert.

Sphingolipide sind komplexe Lipide mit Sphingosin oder einer ähnlichen Base als Grundgerüst. Sie sind wichtige Membrankomponenten pflanzlicher und tierischer Zellen und enthalten drei charakteristische Komponenten: ein Molekül Fettsäure, ein Molekül Sphingosin oder Sphingosin-Derivat und eine polare (Kopf-) Gruppe, die manchmal sehr groß und komplex sein kann. Sphingosin ist einer von etwa 30 langkettigen Aminoalkoholen, die in den Sphingolipiden verschiedener Arten gefunden worden sind. In Säugetieren sind Sphingosin (4-Sphingenin) und Dihydrosphingosin (Sphinganin) die häufigsten Basen der Sphingolipide, in höheren Pflanzen und Hefen Phytosphingosin (4-Hydroxysphinganin) und in marinen Evertebraten doppelt ungesättigte Basen wie 4,8-Sphingadien. Die Sphingosin-Base ist über eine Amidbindung ihrer Aminogruppe mit einer langen gesättigten oder einfach ungesättigten Fettsäure mit 18 bis 26 Kohlenstoffatomen verknüpft. Diese Verbindung, die zwei unpolare Ketten enthält, ist das Ceramid, die charakteristische Grundstruktur aller Sphingolipide. Soweit es nicht ausdrücklich anders angegeben ist, wird im Sinne dieser Anmeldung der Begriff Ceramid gleichbedeutend mit dem Begriff Sphingolipid verwendet.

Die verschiedenen Derivate der Sphingolipide bzw. Ceramide lassen sich weiter in Gruppen nach ihrem Vorkommen und ihren chemisch strukturellen Merkmalen unterteilen. Die häufigsten Sphingolipide in den Geweben höherer Tiere sind die Sphingomyeline, die als polare Gruppen Phosphorylethanolamin oder Phosphorylcholin enthalten. Sie sind Zwitterionen bei pH 7.

Eine zweite Gruppe von Sphingolipiden enthält einen oder mehrere Neutralzucker als polare Gruppe; sie haben daher keine elektrische Ladung und werden daher als neutrale Glycosphingolipide bezeichnet. Die Cerebroside sind die einfachsten Vertreter dieser Gruppe, sie haben als polare Gruppe nur ein Monosaccharid in β-glycosidischer Bindung an ihrer Hydroxyl-Gruppe. In den Cerebrosiden des Gehirns und des Nerversystems kommt D-Galactose vor, sie werden deshalb Galactocerebroside genannt. Cerebroside sind auch in geringen Mengen in nicht-neutralen Geweben vorhanden; hier enthalten Sie meist D-Glucose und werden deshalb Glucocerebroside genannt. Als Sulfatide bezeichnet man Sulfatester der Galactocerebroside, die auch im Himgewebe vorkommen. Cerebroside und Sulfatide enthalten Fettsäuren mit 22 bis 26 Kohlenstoffatomen. Eine verbreitete Fettsäure in Cerebrosiden ist die Cerebronsäure.

Neutrale Glycosphingolipide mit einem Disaccharid werden als Dihexoside bezeichnet. Tri- und Tetrahexoside sind ebenfalls bekannt. In diesen Glycosphingolipiden kommen als Zucker D-Glucose, D-Galactose, N-Acetyl-D-glucosamin und N-Acetyl-D-galactosamin vor. Neutrale Glycosphingolipide sind wichtige Bestandteile der Zelloberflächen in tierischen Geweben. Ihr unpolarer Teil ist in der Lipid-Doppelschicht der Membran verankert, während der polare Teil aus der Oberfläche herausragt.

Eine dritte Gruppe der Sphingolipide sind die sauren Glycosphingolipide, die als Ganglioside bezeichnet werden. Sie enthalten in ihrem Oligosaccharidtell eine oder mehrere Sialinsäuren. Ganglioside kommen besonders in der grauen Himsubstanz vor.

Galactolipide sind hauptsächlich in Pflanzen vorkommende Membranlipide. MGDG (Monogalactosyldiacylglycerol) und DGDG (Digalactosyldiacylglycerol) sind die meist verbreiteten Galactolipide in höheren Pflanzen. Sie kommen vor allem in Plastiden vor und akkumulieren insbesondere in den Thylakoiden der Chloroplasten.

Die erfindungsgemäß verwendeten Vesikel unterscheiden sich ausdrückllich von den ebenfalls in der Kosmetik eingesetzten Liposomen aus Phospholipiden, die hohe Mengen an Phosphatidylcholin enthalten. Phospholipide sind Bestandteil der Zellmembran. Liposome aus Phospholipiden werden daher regelmäßig in Kosmetika als Vesikel für den Transport verschiedener Wirksubstanzen in die Zellen eingesetzt. Es hat sich nun jedoch überraschend herausgestellt, dass die in der erfindungsgemäßen Zusammensetzung verwendeten Vesikel aus Sphingolipiden und/oder Galactolipiden für den Transport von Wirksubstanzen durch die Haut besser geeignet sind als die bekannten Liposomen aus Phospholipiden. Insbesondere bewirken sie einen effektiveren Transport durch die Hornschicht (Stratum Corneum) und in tiefere Hautschichten hinein als dies mit den bekannten Liposomen erreicht wird. Da es sich bei den erfindungsgemäß verwendeten Lipiden um solche handelt, die auch natürlich im Stratum Comeum vorkommen bzw. diesen darin natürlich vorkommenden Lipiden ähnllich sind, bewirkt die Verwendung dieser Lipide beim Auftragen der erfindungsgemäßen kosmetischen Zusammensetzung auf die Haut gleichzeitig eine Festigung der natürlichen Hautbarriere des Stratum Corneum.

Gegenüber Vesikeln aus Phospholipiden zeigte die erfindungsgemäße Zusammensetzung neben einem erhöhten Sauerstofftransport an den gewünschten Wirkort auch eine noch bessere Hautverträglichkeit.

Die Wirkungsweise der Fluorcarbon enthaltenden Zusammensetzung gemäß der Erfindung beruht auf der Abgabe von Sauerstoff an unterversorgtes Gewebe über eine topische Applikation. Eine sinnvolle Anwendung ist auch denkbar für Fettgewebe, das mit Sauerstoff unterversorgt ist, wie auch für arteriosklerotisch bedingte Mangelversorgungen. Wie es weiter unten noch erläutert wird, eignet sich die erfindungsgemäße Zusammensetzung auch zur Sauerstoffversorgung von Diabetikerbeinen und Raucherbeinen. Hierbei handelt es sich um eine periphere Verschlusskrankheit, die zu einer Unterversorgung des Gewebes mit Blut und Sauerstoff führt.

Die Formulierung der erfindungsgemäßen Zusammensetzung als Salben, Cremes, Lotionen und andere wässrige oder alkoholische dermatologische Darreichungsformen erfolgt in Abhängigkeit vom Anwendungszweck, wobei der Fluorcarbongehalt und damit die Sauerstoffverfügbarkeit in breiten Grenzen variiert werden kann. Die Fluorcarbone können vor der Einarbeitung in alle dermatologischen Systeme wie z. B. Gele, Pasten, Puder, Salben, Cremes, Lotionen mit gasförmigem Sauerstoff partiell beladen bzw. gesättigt werden. Bereits die Sättigung mit dem Sauerstoff der atmosphärischen Luft durch die üblicherweise stattfindende Gleichgewichtseinstellung bietet eine höhere Sauerstoffkapazität als alle vergleichbaren bekannten Systeme. Die erfindungsgemäße Zusammensetzung kann auch auf Verbände, Pflaster, Wundabdeckungen und sonstige mit der Haut in Berührung kommende Mittel aufgebracht werden. Sie kann beispielsweise auch als Spray appliziert werden.

Unter dem hier verwendeten Begriff "Fluorcarbone" werden perfluorierte oder hochfluorierte Kohlenstoffverbindungen oder Gemische verstanden, die in der Lage sind, Sauerstoff zu transportieren. Hochgradig fluorierte Kohlenwasserstoffverbindungen sind im Sinne dieser Erfindung solche, bei denen die meisten Wasserstoffatome durch Fluoratome ersetzt sind, wie z. B. die Bis-F-(Alkyl)-ethene, die nachweislich chemisch und biologisch inert und damit untoxisch sind. Dies wird meist dann erreicht, wenn etwa bis zu 90% der Wasserstoffatome durch Fluoratome ersetzt sind. Bevorzugt im Sinne der vorliegenden Erfindung sind Fluorcarbone, bei denen wenigstens 95% der Wasserstoffatome ersetzt sind, bevorzugter 98% und am bevorzugtesten 100%. Es können auch einzelne Fluoratome durch andere Halogenatome wie Brom oder Chlor ersetzt sein.

Eine Vielzahl von sauerstoffbindenden Fluorcarbonen eignet sich für die Verwendung in der erfindungsgemäßen kosmetischen Zusammensetzung. Beispiele für geeignete Fluorcarbone sind aliphatische geradkettige und verzweigte Fluoralkane, mono- oder bizyklische und gegebenenfalls fluoralkylsubstituierte Fluorcycloalkane, perfluorierte aliphatische oder bizyklische Amine, Bis-(Perfluoralkyl)-ethene oder deren Gemische. Besonders bevorzugt sind Perfluordecalin, F-Butyltetrahydrofuran, Perfluortributylamin, Perfluoroctylbromid, Bis-Fluor-(butyl)-ethen und Bis-Fluor-(hexyl)-ethen oder C₆-C₉-Perfluoralkane. Ganz besonders bevorzugt ist Perfluordecalin. Fluorcarbon bzw. Fluorcarbongemische werden erfindungsgemäß in einer Menge von 5-50 Gew.-%, vorzugsweise 10-50 Gew.-%, besonders bevorzugt 15-25 Gew.-% eingesetzt, bezogen auf das Gesamtgewicht der kosmetischen Zusammensetzung.

Bezüglich der Wirkweise der erfindungsgemäß verwendeten Transportvesikel für den Sauerstofftransport in die Haut im Vergleich zu Liposomen aus Phospholipiden nehmen die Erfinder der vorliegenden Anmeldung folgenden Mechanismus an, der jedoch keine Bindungswirkung im Sinne einer Beschränkung dieser Anmeldung haben soll: Die Vesikel aus Sphingolipiden und/oder Galactolipiden der erfindungsgemäßen Zusammensetzung dringen zunächst in die oberen Hautschichten ein, wo sie dann aber mit diesen Hautschichten, insbesondere dem Stratum Comeum verschmelzen und die Wirksubstanz freigeben. Die Vesikel wirken Okklusiv, d. h. sie bilden eine Barriere gegen den Rücktransport der freigesetzten Wirksubstanz bzw. des Sauerstoffs. Der Sauerstoff wandert bzw. diffundiert weiter in tiefere Hautschichten unterhalb des Stratum Comeum, wo er dann seine vorteilhafte Wirkung entfalten kann. Im Gegensatz dazu bleiben Liposome aus Phospholipiden im wesentlichen stabil und penetrieren bzw. durchwandem die Haut als Vesikel. Die Freisetzung der Wirksubstanz aus den Liposomen ist aufgrund ihrer hohen Stabilität geringer als bei den erfindungsgemäß verwendeten Vesikeln, wodurch sich auch die verbesserte Wirkung der erfindungsgemäß verwendeten Vesikel erklärt.

In einer bevorzugten Ausführungsform der erfindungsgemäßen kosmetischen Zusammensetzung sind die Sphingolipide bzw. Ceramide unter neutralen Glycosphingolipiden, wie Cerebrosiden, Galactocerebrosiden, Glucocerebrosiden und Sulfatiden ausgewählt.

In einer weiteren bevorzugten Ausführungsform der erfindungsgemäßen kosmetischen Zusammensetzung sind die Galactolipide unter Monogalactosyldiacylglycerol und Digalactosyldiacylglycerol ausgewählt.

In einer weiteren bevorzugten Ausführungsform der erfindungsgemäßen kosmetischen Zusammensetzung sind die Vesikel in der Zusammensetzung als Vesikel mit Lipiddoppelschicht-Membranen enthalten.

Die erfindungsgemäße kosmetische Zusammensetzung kann mit Vorteil weitere dermatologisch geeignete Zusatzstoffe, Emulgiermittel, Konservierungsmittel, Trägersubstanzen, Lösungsvermittler, Verdicker und/oder Stabilisatoren enthalten.

Besonders bevorzugt enthält die erfindungsgemäße kosmetische Zusammensetzung weiterhin 5-20 Gew.-% Öle und/oder Wachse, vorzugsweise pflanzliche Öle und/oder pflanzliche Wachse. Ganz besonders bevorzugt ist Jojoba-Öl, welches ein besonders angenehmes Hautgefühl beim Auftragen der kosmetischen Zusammensetzung vermittelt. Ebenfalls geeignet ist Sonnenblumenöl. Zweckmäßigerweise werden die Sphingolipide und/oder Galactolipide in der Form einer Lösung in Öl in die erfindungsgemäße kosmetische Zusammensetzung eingebracht. Eine solche Öl-Lösung enthält zweckmäßigerweise 10-20 Gew.-% Sphingolipide und/oder Galactolipide in Öl.

Gemäß einer weiteren bevorzugten Ausführungsform enthält die erfindungsgemäße kosmetische Zusammensetzung weiterhin 10-50 Gew.-% Alkohole, vorzugsweise Glycerin, Ethanol und/oder Glycole, wie 1,2-Propylenglycol, 1,3-Butylenglycol oder 1,2-Pentylenglycol. Glycerin ist in der erfindungsgemäßen kosmetischen Zusammensetzung vorzugsweise in einer Menge von 10-20 Gew.-%, besonders bevorzugt von 14-18 Gew.-% enthalten. Glycerin wirkt feuchtigkeitsspendend für die Haut und stabilisiert die erfindungsgemäße Zusammensetzung. Ethanol und/oder 1,2-Propylenglycol, 1,3-Butylenglycol bzw. 1,2-Pentylenglycol werden vorzugsweise in einer Menge von 5-30 Gew.-%, besonders bevorzugt 15-25 Gew.-% eingesetzt. Die Glycole wirken feuchtigkeitsspendend und als Lösungsvermittler. Weiterhin besitzen sie ein antimikrobielles Wirkspektrum.

In einer weiteren bevorzugten Ausführungsform enthält die erfindungsgemäße kosmetische Zusammensetzung weiterhin 0,5 bis 5 Gew.-%, besonders bevorzugt 1,0 bis 3,0 Gew.-% eines Polyethylenglycol-Fettsäureglycerides, vorzugsweise eines Fettsäureglycerides von Polyethylenglycol-25 bis Polyethylenglycol-75. Besonders bevorzugt ist das Fettsäureglycerid ein Shea-Butter-Glycerid. Alternativ eignet sich aber auch Kokosnuß-Glycerid oder andere Ölglyceride.
Die Polyethylenglycol-Fettsäureglyceride bewirken eine sterische Abschirmung der Transportvesikel in der erfindungsgemäßen Zusammensetzung und führen somit zu einer Stabilisierung der Vesikelsuspension.
Je nach Zusammensetzung kann es zweckmäßig oder notwendig sein, der erfindungsgemäßen kosmetischen Zusammensetzung weiterhin Konservierungsmittel und/oder Verdickungsmittel zuzusetzen. Konservierungsmittel können dabei in einer Menge von 0,01 bis 1 Gew.-%, Verdickungsmittel in einer Menge von 0,05 bis 2 Gew.-% zugesetzt werden. Besonders bevorzugt sind jedoch konservierungsmittelfreie Zusammensetzungen.

In einer weiteren Ausführungsform der kosmetischen bzw. pharmazeutischen Zusammensetzung der vorliegenden Erfindung enthält die Zusammensetzung weiterhin natürliches oder synthetisches Capsaicin (Nonylsäurevanillylamid), vorzugsweise in einer Menge von 0,1 bis 1 Gew.-%, besonders bevorzugt in einer Menge von 0,2 bis 0,6 Gew.-%, und/oder Nicotinsäure und/oder Nicotinsäureamid und/oder Nicotinsäureester, vorzugsweise in einer Menge von 0,5 bis 5 Gew.-%, besonders bevorzugt in einer Menge von 0,5 bis 3 Gew.-%. Aufgrund der analgetischen und durchblutungsfördernden Wirkung der vorgenannten Wirkstoffe kann diese Ausführungsform der erfindungsgemäßen Zusammensetzung mit Vorteil zur Regenerierung und Verbesserung der Zustände von Diabetikerbeinen und Raucherbeinen eingesetzt werden. Bei Diabetiker- und Raucherbeinen ist die Sauerstoffversorgung gegenüber dem Normalzustand stark eingeschränkt bzw. gestört. Durch die Anwendung der erfindungsgemäßen Zusammensetzung kann dieser Sauerstoffmangel wenigstens teilweise ausgeglichen und die Begleitbeschwerden können gelindert werden. Die gleichzeitige Auslieferung der vorgenannten Wirkstoffe dieser Ausführungsform der Erfindung führt zur Erzeugung von Wärme, was die Regeneration fördert.

Die Erfindung wird nun anhand der nachfolgenden Beispiele näher erläutert.

### BEISPIEL 1

Eine erfindungsgemäß besonders bevorzugte kosmetische Zusammensetzung für die Hautbehandlung enthält:

| | |
|---|---|
| 20,0 Gew.-% | 1,2-Propylenglycol, |
| 16,0 Gew.-% | Glycerin, |
| 10,0 Gew.-% | Sphingolipid-Öl/Wachs-Lösung (15 Gew.-% Glycosphingolipide in Jojoba-Öl), |
| 2,0 Gew.-% | Polyethylenglycol-75-Shea-Butter-Glyceride, |
| 0,2 Gew.-% | Xanthan Gum, |
| 20,0 Gew.-% | Perfluordecalin, |
| 0,05 Gew.-% | Konservierungsmittel (Euxyl K702^{®}, Schülke&Mayr, DE ) |
| ad 100 Gew.-% | Wasser. |

Zur Herstellung dieser Zusammensetzung werden 1,2-Propylenglycol und Glycerin sorgfältig homogen gemischt und in einem Becherglas vorgelegt (klare, farblose, leicht viskose Lösung). Unter Turraxieren (= Homogenisieren unter Verwendung eines Turrax-Homogenisators bei 10.000 U/min) werden nacheinander die Sphingolipid-Öl/Wachs-Lösung, das Polyethylenglycol-75-Shea-Butter-Glycerid und Xanthan Gum eingearbeitet. Die viskose, hellbeige Lösung wird anschließend für weitere 20 min. nachhomogenisiert. Unter weiterem stetigem Turraxieren (ca. 10.000 U/min) wird das Perfluordecalin eingearbeitet und solange weiter homogenisiert, bis eine weiße, homogene Emulsion entsteht. Nun wird unter Turraxieren (ca. 10.000 U/min) das Wasser zugefügt. Die weiße Lösung wird anschließend für weitere 20 min. nachhomogenisiert und abschließend konserviert. Der pH-Wert der Emulsion wird erforderlichenfalls mit Natronlauge auf pH 4,5 bis 6,5 eingestellt. Die gemessene Vesikelgröße in der kosmetischen Zusammensetzung betrug 150 bis 300 nm.

### BEISPIEL 2

Eine weitere erfindungsgemäße Zusammensetzung für Diabetiker- und Raucherbeine enthält:

| | |
|---|---|
| 20,0 Gew.-% | 1,2-Propylenglycol, |
| 16,0 Gew.-% | Glycerin, |
| 10,0 Gew.-% | Sphingolipid-Öl/Wachs-Lösung (15 Gew.-% Glycosphingolipide in Sonnenblumenöl), |
| 2,0 Gew.-% | Polyethylenglycol-75-Shea-Butter-Glyceride, |
| 0,2 Gew.-% | Xanthan Gum, |
| 0,5 Gew.-% | Nonylsäurevanillylamid, |
| 20,0 Gew.-% | Perfluordecalin, |
| 0,05 Gew.-% | Konservierungsmittel (Euxyl K702^{®}, Schülke&Mayr, DE ), |
| ad 100 Gew.-% | Wasser. |

Zur Herstellung dieser Zusammensetzung werden 1,2-Propylenglycol und Glycerin sorgfältig homogen gemischt und in einem Becherglas vorgelegt. In dieser Mischung wird das Nonylsäurevanillylamid vollständig gelöst (klare, fast farblose, leicht viskose Lösung). Unter Turraxieren (= Homogenisieren unter Verwendung eines Turrax-Homogenisators bei 10.000 U/min) werden nacheinander die Sphingolipid-Öl/Wachs-Lösung, das Polyethylenglycol-75-Shea-Butter-Glycerid und Xanthan Gum eingearbeitet. Die viskose, hellbeige Lösung wird anschließend für weitere 20 min. nachhomogenisiert. Unter weiterem stetigem Turraxieren (ca. 10.000 U/min) wird das Perfluordecalin eingearbeitet und solange weiter homogenisiert, bis eine weiße, homogene Emulsion entsteht. Nun wird unter Turraxieren (ca. 10.000 U/min) das Wasser zugefügt. Die weiße Lösung wird anschließend für weitere 20 min. nachhomogenisiert und abschließend konserviert. Der pH-Wert der Emulsion wird erforderlichenfalls mit Natronlauge auf pH 4,5 bis 6,5 eingestellt. Die gemessene Vesikelgröße in der kosmetischen Zusammensetzung betrug 200 bis 450 nm.

### BEISPIEL 3

Die Wirksamkeit der erfindungsgemäßen kosmetischen Zusammensetzung gemäß Beispiel 1 wurde hinsichtlich des Sauerstofftransports durch die Haut an sechs gesunden weiblichen Probanden im Alter zwischen 20 und 50 Jahren untersucht. Aus einer früheren Studie von C. Artmann et al. (SÖFW Journal 15, 1993, S. 6-8), in welcher der Sauerstoffpartialdruck (pO₂) an 361 Probanden festgestellt worden war, wurde gezeigt, daß männliche Probanden deutlich niedrigere pO₂-Werte aufwiesen als weibliche Probanden. Im vorliegenden Experiment wurden die Messungen der transkutanen Sauerstoffpartialdrücke (pO₂) an der Innenseite des Unterarms mit einer polarographischen Sonde (Clark-Methode) durchgeführt. Die transkutanen Sauerstoffpartialdrücke wurden als "mm Hg" mit einem OMED pO₂-Analysierer (Bretzfeld, Deutschland) aufgezeichnet. Zur Erzeugung einer örtlichen Arterialisation wurde der Sensor auf eine konstante Temperatur von 42°C (leicht hyperthermisch) erwärmt, wodurch der Sensor eine quantitative Bestimmung des Sauerstoffpartialdrucks im Bereich des arterialisierten Hautgewebes erlaubt. Der Ausgangswert des Sauerstoffpartialdrucks ohne Behandlung mit der erfindungsgemäßen kosmetischen Zusammensetzung wurde nach 20 Minuten gemessen. Anschließend wurden auf jeweils einen Arm der Probanden 5 µl der erfindungsgemäßen kosmetischen Zusammensetzung gemäß Beispiel 1 auf einen Hautbereich von 1 cm² aufgetragen und der jeweils andere Arm als Kontrolle unbehandelt gelassen. Das Experiment wurde um 9.00 Uhr vormittags begonnen, und über einen Zeitraum von sechs Stunden wurde in Abständen von jeweils 1,5 Stunden die Veränderung des Sauerstoffpartialdrucks gemessen. Die Ergebnisse sind in der nachfolgenden Tabelle 1 aufgeführt.

Die Daten zeigen, daß der transkutane Sauerstoffdruck stark tageszeitabhängig ist. Während der Mittagszeit wurden in allen Probanden sehr niedrige Werte des Sauerstoffpartialdrucks beobachtet. Am Nachmittag stieg der Sauerstoffpartialdruck bei allen Probanden wieder an. Die Ergebnisse in Tabelle 1, welche Durchschnittswerte der Messungen an allen sechs Probanden wiedergeben, zeigen eine deutliche Erhöhung der Sauerstoffkonzentration in den tieferen Hautschichten bereits nach 1,5 Stunden. Die signifikante Erhöhung des Sauerstoffgehalts in den mit der erfindungsgemäßen Zusammensetzung behandelten Hautpartien gegenüber der unbehandelten Haut blieb über den Zeitraum von sechs Stunden des Experiments im wesentlichen gleich. Der Unterschied im Sauerstoffpartialdruck zwischen behandelter und unbehandelter Haut betrug im Durchschnitt etwa 9 mm Hg, was einem Wert entspricht, der durch die Inhalation von reinem Sauerstoff erzielt werden kann. Bereits durch den einmaligen Auftrag der erfindungsgemäßen kosmetischen Zusammensetzung konnte somit eine deutliche Erhöhung der Sauerstoffkonzentration in der Haut erzielt werden.

**TABELLE 1**

| Zeit [h] | Tageszeit | unbehandelt | behandelt |
|---|---|---|---|
| | | pO₂ [mm Hg] | pO₂ [mm Hg] |
| 0 | 9:00 | 86 | 86 |
| 1,5 | 10:30 | 83 | 91 |
| 3 | 12:00 | 79 | 88 |
| 4,5 | 13:30 | 72 | 81 |
| 6 | 15:00 | 77 | 86 |

## Patentansprüche

1. Kosmetische Zusammensetzung zur Unterstützung des Sauerstofftransports in die Haut, wobei die Zusammensetzung für den Sauerstofftransport einen Anteil an membranbildenden Lipiden und einen Anteil an Fluorcarbon oder Fluorcarbongemisch enthält,
**dadurch gekennzeichnet, dass** die Zusammensetzung
1 bis 50 Gew.-% membranbildende Sphingolipide und/oder Galactolipide und 5 bis 50 Gew.-% mit Sauerstoff beladenes Fluorcarbon oder Fluorcarbongemisch enthält, wobei die Sphingolipide und/oder Galactolipide als Transportvesikel für das mit Sauerstoff beladene Fluorcarbon oder Fluorcarbongemisch dienen.

2. Kosmetische Zusammensetzung nach Anspruch 1, **dadurch gekennzeichnet, dass** die Sphingolipide unter neutralen Glycosphingolipiden, wie Cerebrosiden, Galactocerebrosiden, Glucocerebrosiden und Sulfatiden, ausgewählt sind.

3. Kosmetische Zusammensetzung nach einem der vorausgegangenen Ansprüche, **dadurch gekennzeichnet, dass** die Galactolipide unter Monogalactosyldiacylglycerol und Digalactosyldiacylglycerol ausgewählt sind.

4. Kosmetische Zusammensetzung nach einem der vorausgegangenen Ansprüche, **dadurch gekennzeichnet, dass** die Vesikel in der Zusammensetzung als Vesikel mit Lipiddoppelschicht-Membranen enthalten sind.

5. Kosmetische Zusammensetzung nach einem der vorausgegangenen Ansprüche, **dadurch gekennzeichnet, dass** die Vesikel in der Zusammensetzung als Nanopartikel bzw. Nanoemulsion, vorzugsweise mit Lipideinzelschicht-Membranen, enthalten sind.

6. Kosmetische Zusammensetzung nach einem der vorausgegangenen Ansprüche, **dadurch gekennzeichnet, dass** das Fluorcarbon Perfluordecalin ist.

7. Kosmetische Zusammensetzung nach einem der vorausgegangenen Ansprüche, **dadurch gekennzeichnet, dass** die Zusammensetzung weiterhin 5 bis 20 Gew.-% Öle und/oder Wachse, vorzugsweise pflanzliche Öle und/oder pflanzliche Wachse, besonders bervorzugt Jojoba-Öl enthält.

8. Kosmetische Zusammensetzung nach einem der vorausgegangenen Ansprüche, **dadurch gekennzeichnet, dass** die Zusammensetzung weiterhin natürliches oder synthetisches Capsaicin (Nonylsäurevanillylamid), vorzugsweise in einer Menge von 0,1 bis 1 Gew.-%, besonders bevorzugt in einer Menge von 0,2 bis 0,6 Gew.-%, und/oder Nicotinsäure und/oder Nicotinsäureamid und/oder Nicotinsäureester, vorzugsweise in einer Menge von 0,5 bis 5 Gew.-%, besonders bevorzugt in einer Menge von 0,5 bis 3 Gew.-% enthält.

9. Kosmetische Zusammensetzung nach einem der vorausgegangenen Ansprüche, **dadurch gekennzeichnet, dass** die Zusammensetzung weiterhin 10 bis 50 Gew.-% Alkohole, vorzugsweise Glycerin, Ethanol und/oder Glycole, wie 1,2-Propylenglycol, 1,3-Butylenglycol oder 1,2-Pentylenglycol enthält.

10. Kosmetische Zusammensetzung nach einem der vorausgegangenen Ansprüche, **dadurch gekennzeichnet; dass** die Zusammensetzung weiterhin 0,5 bis 5 Gew.-% eines Polyethylenglycol-Fettsäureglycerides, vorzugsweise eines Fettsäureglycerides von Polyethylenglycol-25 bis Polyethylenglycol-75, enthält.

11. Kosmetische Zusammensetzung nach einem der vorausgegangenen Ansprüche, **dadurch gekennzeichnet, dass** die Zusammensetzung weiterhin Konservierungsmittel in einer Menge von 0,01 bis 1,0 Gew.-% und/oder Verdickungsmittel in einer Menge von 0,05 bis 2,0 Gew.-% enthält.

12. Kosmetische Zusammensetzung nach einem der vorausgegangenen Ansprüche, **dadurch gekennzeichnet, dass** die Zusammensetzung
| | |
|---|---|
| 5 bis 30 Gew.-%, vorzugsweise 15 bis 25 Gew.-% | 1,2-Propylenglycol, |
| 10 bis 20 Gew.-%, vorzugsweise 14 bis 18 Gew.-% | Glycerin, |
| 5 bis 20 Gew.-%, vorzugsweise 7 bis 15 Gew.-% | Sphingolipid-Öl/Wachs-Lösung, |
| 0,5 bis 5 Gew.-%, vorzugsweise 1 bis 3 Gew.-% | Polyethylenglycol-75-Shea-Butter-Glycerid, |
| 10 bis 50 Gew.-%, vorzugsweise 15 bis 25 Gew.-% | Perfluordecalin und |
ad 100 Gew.-% Wasser
enthält, wobei die Sphingolipid-Öl/Wachs-Lösung im wesentlichen aus einer Lösung von 10 bis 20 Gew.-% Ceramiden und/oder Glycosphingolipiden in Ölen und/oder Wachsen, vorzugsweise pflanzlichen Ölen und/oder pflanzlichen Wachsen, besonders bevorzugt Jojoba-Öl besteht.

## Claims

1. A cosmetic composition to assist oxygen transport into the skin, wherein the composition contains for transporting the oxygen a portion consisting of membrane-forming lipids and a portion consisting of a fluorocarbon or fluorocarbon mixture,
**characterized in that** the composition contains
1% to 50% by weight of membrane-forming sphingolipids and/or galactolipids; and
5% to 50% by weight of a fluorocarbon or fluorocarbon mixture charged with oxygen,
said sphingolipids and/or galactolipids acting as transport vesicles for the fluorocarbon or fluorocarbon mixture charged with oxygen.

2. A cosmetic composition according to claim 1, **characterized in that** the sphingolipids are selected from neutral glycosphingolipids, such as cerebrosides, galactocerebrosides, glucocerebrosides and sulphatides.

3. A cosmetic composition according to one of the preceding claims, **characterized in that** the galactolipids are selected from monogalactosyldiacylglycerol and digalactosyldiacylglycerol.

4. A cosmetic composition according to one of the preceding claims, **characterized in that** the vesicles are contained in the composition as a vesicle with a double lipid layer membrane.

5. A cosmetic composition according to one of the preceding claims, **characterized in that** the vesicles in the composition are present as nanoparticles or a nanoemulsion, preferably with single lipid layer membranes.

6. A cosmetic composition according to one of the preceding claims, **characterized in that** the fluorocarbon is perfluorodecalin.

7. A cosmetic composition according to one of the preceding claims, **characterized in that** the composition further contain 5% to 20% by weight of oils and/or waxes, preferably vegetable oils and/or vegetable waxes, more preferably jojoba oil.

8. A cosmetic composition according to one of the preceding claims, **characterized in that** the composition further contains natural or synthetic capsaicin (nonylic acid vanillylamide), preferably in an amount of 0.1 % to 1% by weight, particularly preferably in an amount of 0.2% to 0.6% by weight, and/or nicotinic acid and/or nicotinamide and/or nicotinic acid ester, preferably in an amount of 0.5% to 5% by weight, particularly preferably in an amount of 0.5% to 3% by weight.

9. A cosmetic composition according to one of the preceding claims, **characterized in that** the composition further contains 10% to 50% by weight of alcohols, preferably glycerin, ethanol and/or glycols, such as 1,2-propylene glycol, 1,3-butylene glycol or 1,2-pentylene glycol.

10. A cosmetic composition according to one of the preceding claims, **characterized in that** the composition further contains 0.5% to 5% by weight of a polyethylene glycol fatty acid glyceride, preferably a fatty acid glyceride of polyethylene glycol 25 to polyethylene glycol 75.

11. A cosmetic composition according to one of the preceding claims, **characterized in that** the composition further contains preservatives in an amount of 0.01% to 1.0% by weight and/or thickening agents in an amount of 0.05% to 2.0% by weight.

12. A cosmetic composition according to one of the preceding claims, **characterized in that** the composition contains:
5% to 30% by weight, preferably 15% to 25% by weight of 1,2-propyleneglycol;
10% to 20% by weight, preferably 14% to 18% by weight of glycerin;
5% to 20% by weight, preferably 7% to 15% by weight of sphingolipid-oil/wax solution;
0.5% to 5% by weight, preferably 1% to 3% by weight of polyethyleneglycol 75 shea butter glyceride;
10% to 50% by weight, preferably 15% to 25% by weight of perfluordecalin; and qs 100% by weight of water,
wherein the sphingolipid-oil/wax solution substantially comprises of a solution of 10% to 20% by weight of ceramides and/or glycosphingolipids in oils and/or waxes, preferably vegetable oils and/or vegetable waxes, more preferably jojoba oil.

## Revendications

1. Composition cosmétique destinée à favoriser la pénétration de l'oxygène dans la peau, la composition destinée à la pénétration de l'oxygène contenant un pourcentage de lipides membranaires et un pourcentage de fluorure de carbone ou d'un composé du fluorure de carbone,
**caractérisée en ce que** la composition contient
1 à 50% en poids de galactolipides et sphingolipides membranaires et
5 à 50% en poids de fluorure de carbone, ou d'un composé du fluorure de carbone, chargé d'oxygène, les sphingolipides et/ou les galactolipides servant de vésicules de pénétration du fluorure carbone, ou d'un composé du fluorure carbone, chargé d'oxygène.

2. Composition cosmétique selon la revendication 1, **caractérisée en ce que** les sphingolipides sont choisis parmi les glycosphingolipides neutres tels que les cérébrosides, les galactocérébrosides, les glucocérébrosides et les sulfatides.

3. Composition cosmétique selon l'une des revendications précédentes,
**caractérisée en ce que** les galactolipides sont choisis parmi le monogalactosyldiacylglycerol et le digalactosyldiacylglycerol.

4. Composition cosmétique selon l'une des revendications précédentes,
**caractérisée en ce que** les vésicules sont contenues dans la composition sous la forme de vésicules comportant des membranes à double couche lipidique.

5. Composition cosmétique selon l'une des revendications précédentes,
**caractérisée en ce que** les vésicules sont contenues dans la composition sous la forme de nanoparticules respectivement d'une nanoémulsion, comportant avantageusement des membranes à simple couche lipidique.

6. Composition cosmétique selon l'une des revendications précédentes,
**caractérisée en ce que** le fluorure de carbone est du perfluorodécaline.

7. Composition cosmétique selon l'une des revendications précédentes,
**caractérisée en ce que** la composition contient en outre 5 à 20% en poids d'huiles et/ou de cires, avantageusement d'huiles végétales et/ou de cires végétales, notamment de préférence d'huile de jojoba.

8. Composition cosmétique selon l'une des revendications précédentes,
**caractérisée en ce que** la composition contient en outre de la capsaïcine (vanillylamide de l'acide nonylique) naturelle ou de synthèse, avantageusement dans une quantité de 0,1 à 1% en poids, notamment de préférence dans une quantité de 0,2 à 0,6% en poids, et/ou de l'acide nicotinique et/ou d'un amide de l'acide nicotinique, et/ou d'un ester de l'acide nicotinique, avantageusement dans une quantité de 0,5 à 5% en poids, notamment de préférence dans une quantité de 0,5 à 3% en poids.

9. Composition cosmétique selon l'une des revendications précédentes,
**caractérisée en ce que** la composition contient en outre 10 à 50% en poids d'alcool, avantageusement de glycérine, d'éthanol et/ou de glycol, tels que du 1,2-propylène-glycol, 1,3-butylène-glycol ou 1,2-pentylène-glycol.

10. Composition cosmétique selon l'une des revendications précédentes,
**caractérisée en ce que** la composition contient en outre 0,5 à 5% en poids d'un glycéride d'acide gras de polyéthylène-glycol, avantageusement d'un glycéride d'acide gras de polyéthylène-glycol 25 à polyéthylène-glycol 75.

11. Composition cosmétique selon l'une des revendications précédentes,
**caractérisée en ce que** la composition contient en outre des agents conservateurs dans une quantité de 0,01 à 1,0% en poids et/ou des épaississants dans une quantité de 0,05 à 2,0% en poids.

12. Composition cosmétique selon l'une des revendications précédentes,
**caractérisée en ce que** la composition contient
- 5 à 30% en poids, avantageusement 15 à 25% en poids, de 1,2-propylène-glycol,
- 10 à 20% en poids, avantageusement 14 à 18% en poids, de glycérine,
- 5 à 20% en poids, avantageusement 7 à 15% en poids, de solution d'huile ou de cire de sphingolipide,
- 0,5 à 5% en poids, avantageusement 1 à 3% en poids, de glycéride de beurre karité de polyéthylène-75,
- 10 à 50% en poids, avantageusement 15 à 25% en poids, de perfluorodécaline et le tout rapporté à 100% en poids d'eau,
la solution d'huile ou de cire de sphingolipide étant constituée essentiellement d'une solution de 10 à 20% en poids de céramides et/ou de glycosphingolipides dans des huiles et/ou des cires, avantageusement des huiles végétales et/ou des cires végétales, notamment de préférence de l'huile de jojoba.
